# EUROPÄISCHE PATENTSCHRIFT

(11) **EP 2 965 709 B1**
(45) Veröffentlichungstag und Bekanntmachung des Hinweises auf die Patenterteilung: **27.12.2017**
(21) Anmeldenummer: 14176040.5
(22) Anmeldetag: 08.07.2014
(51) Int. Cl.: A61C 1/18, A61C 1/00, A61B 1/247, A61B 17/00, A61C 1/08

(54) **Kupplungsvorrichtung zur lösbaren Verbindung eines medizinischen, insbesondere zahnärztlichen, Handstücks mit einer Antriebseinheit oder einem Versorgungsschlauch**
Coupling device for detachable connection of a medical instrument, in particular a dental handpiece with a drive unit or a supply hose
Dispositif d'accouplement pour la connexion amovible d'une pièce à main médicale, en particulier dentaire, dotée d'une unité d'entraînement ou d'un tuyau flexible d'alimentation

(43) Veröffentlichungstag der Anmeldung: 13.01.2016
(73) Patentinhaber: W & H Dentalwerk Bürmoos GmbH, 5111 Bürmoos (AT)
(72) Erfinder: Mangelberger, Michael, 5113 St. Georgen (AT); Pruckner, Christian, 1190 Wien (AT)

(56) Entgegenhaltungen:
- EP-A1- 1 321 108
- EP-A1- 2 581 061
- AT-B- 389 633
- DE-A1-102006 051 511

## Beschreibung

Die vorliegende Erfindung bezieht sich auf eine Kupplungsvorrichtung zur lösbaren Verbindung eines medizinischen, insbesondere zahnärztlichen, Handstücks mit einer Antriebseinheit oder einem Versorgungsschlauch nach dem Oberbegriff des Anspruchs 1.

Derartige Kupplungsvorrichtungen dienen zur Übertragung von Signalen, Daten, Energie, Licht, einer Antriebsbewegung und/ oder eines Arbeitsmediums, insbesondere eines Fluids, zwischen zwei medizinischen, insbesondere zahnärztlichen, Vorrichtungen. Eine der Vorrichtungen ist hierbei vorzugsweise als zahnärztliches Hand- oder Winkelstück zum Anschluss medizinischer, insbesondere zahnärztlicher, Werkzeuge, welche bevorzugt zur Bearbeitung von hartem oder weichem Gewebe oder zum Einbringen von Implantaten dienen, ausgebildet. Des Weiteren werden unter dem Begriff Handstücke insbesondere gerade, gebogene oder pistolenförmige Handstücke als auch Teile von Handstücken, Adapter, Diagnosegeräte und Lichtsonden verstanden. Bevorzugt werden die Hand- oder Winkelstücke mittels der Kupplungsvorrichtung, insbesondere mittels eines ersten Kupplungsteils der Kupplungsvorrichtung, mit dem zweiten Kupplungsteil, welches vorzugsweise an einer medizinischen, insbesondere zahnärztlichen, Antriebseinheit oder einem Versorgungsschlauch angeordnet ist, verbunden, um die medizinischen Werkzeuge durch einen in der Antriebseinheit angeordneten Elektromotor anzutreiben. Während des Betriebs der medizinischen Werkzeuge sind diese sowie die Hand- oder Winkelstücke mit Arbeitsmedien, wie zum Beispiel Sprayluft und/ oder Spraywasser zur Kühlung oder elektrischer Energie, zu versorgen. Diese Medien werden insbesondere von einer zahnärztlichen Einheit bereit gestellt und mittels einer Versorgungsleitung über die Kupplungsvorrichtung der Antriebseinheit oder dem Hand- oder Winkelstück zugeführt.

Zur Übertragung von Daten und Energie weisen die Kupplungsvorrichtungen oftmals elektrische Kontakte oder Induktionsspulen auf. Die Spulen sind hierbei bevorzugt im Inneren der Kupplungsteile der Kupplungsvorrichtung, insbesondere in einer Hülsenwand, in einem Kupplungszapfen, oder hinter einer Kupplungszapfenbasis der Kupplungsvorrichtung, angeordnet.

Eine derartige Kupplungsvorrichtung mit einem ersten und zweiten Kupplungselement zum Versorgen des Handstücks mit einer Antriebsbewegung, Arbeitsmedien sowie mit Daten und Energie ist insbesondere aus der EP 2 581 061 A1 und der DE 10 2006 051 511 A1 bekannt. In beiden Schriften weist die Kupplungsvorrichtung ein Positionierungselement an einem Kupplungselement auf, das in eine Vertiefung an dem anderen Kupplungselement einführbar ist, um beide Kupplungselemente in einer definierten Winkelposition um deren gemeinsame Achse zueinander zu positionieren. In dem Positionierungselement und der Vertiefung sind elektrische Kontakte oder Induktionsspulen zur Signal-, Daten- und Energieübertragung vorgesehen.

Die Patentschrift AT 389 633 B offenbart eine Schaltvorrichtung, die es ermöglicht, mit einem einzigen Motor unterschiedliche Handstück, die wahlweise eine oder keine Lichtquelle haben, zu betreiben. An dem motorseitigen Kupplungselement sind dazu eine Lichtquelle und stiftförmige elektrische Kontakte vorgesehen.

Die Patentanmeldung EP 1 321 108 A1 offenbart ein zahnärztliches Arbeitsgerät, mit einem Handstück zur Aufnahme eines angetriebenen Werkzeugs, wobei das Handstück durch einen Leitungen enthaltenden Schlauch mit einem Versorgungsgerät drehbar verbunden ist.

An einem der beiden Kupplungselemente der Kupplungsvorrichtung ist ein Positionierungselement angeordnet, um beide Kupplungselemente in einer definierten Winkelposition zueinander zu positionieren. Zur Speicherung von instrumentenbezogenen Daten ist in dem Positionierungselement eine Speichereinheit angeordnet. Die Übertragung der Daten von der Speichereinheit auf das andere Kupplungselement erfolgt hierbei bevorzugt drahtgebunden mittels eines ersten elektrischen Kontakts in dem Positionierungselement und eines zweiten elektrischen Kontakts in einer Vertiefung an dem anderen Kupplungselement. Zur drahtlosen Übertragung von Energie, vorzugsweise für eine Lichtquelle in dem Handstück, weist das Positionierungselement und die Vertiefung des Weiteren jeweils eine Induktionsspule auf.

Als nachteilig dieser Ausgestaltung der Kupplungsvorrichtung erweist sich die Übertragung der instrumentenbezogenen Daten von der Speichereinheit mittels eines elektrischen Kontakts und die Energieübertragung für ein elektrisches Bauteil mittels einer Induktionsspule in dem Positionierungselement der Kupplungsvorrichtung.

Bei einer gleichzeitigen Energie- und Datenübertragung ist es erforderlich alle im Bereich der elektrischen Kontakte und der Induktionsspulen liegenden Bauteile aus nicht elektrisch leitenden Materialen auszubilden, um den Energie- und Datentransfer nicht durch die in den elektrisch leitenden Bauteilen erzeugten Gegenfelder zu gefährden. Bei der im Stand der Technik bekannten Anordnung von einem elektrischen Kontakt, einer elektrischen Leitung sowie einer Induktionsspule in dem Positionierungselement besteht somit die Gefahr, dass durch die elektrisch leitenden Bauteile elektrische oder magnetische Gegenfelder erzeugt werden, welche die Energie- und Datenübertragung zwischen den beide Kupplungselementen gefährden.

Auch eine Abschirmung der elektrisch leitenden Bauteile für die Energie- und Datenübertragung erweist sich auf Grund von Mehrkosten für die aufwendige Schirmung, als auch auf Grund des damit verbundenen größeren Platzbedarfs für die Daten- und Energieübertragung als nachteilig.

Einen weiteren Nachteil der aus dem Stand der Technik bekannten Ausführungsform stellt die beschränkte Daten- und Energieübertragung dar. Die Anordnung der elektrischen Kontakte und der Induktionsspulen in dem Positionierungselement und der Vertiefung ermöglicht zwar auf Grund der genormten Baugröße des Positionierungselements bereits bestehende Kupplungsvorrichtungen, insbesondere zahnärztliche Handstücke, zum Senden und Empfangen von Daten und Energie auszubilden, jedoch ist eine Energie- und Datenübertragung aus Platzgründen nur begrenzt möglich. Insbesondere können mehrere Daten und Signale, wie zum Beispiel Informationen zur Wurzelkanallänge, Sensordaten oder Identifikationssignale, mittels der bekannten Kupplungsvorrichtung nur seriell von dem einen Kupplungselement auf das andere Kupplungselement der Kupplungsvorrichtung übertragen werden.

Der vorliegenden Erfindung liegt daher die Aufgabe zugrunde eine Kupplungsvorrichtung zur lösbaren Verbindung eines medizinischen, insbesondere zahnärztlichen, Handstücks mit einer Antriebseinheit oder einem Versorgungsschlauch zu schaffen, die es insbesondere ermöglicht Signale, Daten und/ oder Energie sicher und für mehrere elektronische Komponenten von dem einen Kupplungselement auf das andere Kupplungselement der Kupplungsvorrichtung zu übertragen.

Diese Aufgabe wird durch eine Kupplungsvorrichtung gemäß dem Anspruch 1 gelöst.

Die Kupplungsvorrichtung zur lösbaren Verbindung eines medizinischen, insbesondere zahnärztlichen, Handstücks mit einer Antriebseinheit oder einem Versorgungsschlauch zur Übertragung von Signalen, Daten und/ oder Energie, einer Antriebsbewegung, eines Arbeitsmediums und/ oder von Licht zwischen dem Handstück und der Antriebseinheit oder dem Versorgungsschlauch umfasst: ein erstes und zweites Kupplungselement, wobei eines der beiden Kupplungselemente als Kupplungsausnehmung ausgebildet ist, in die ein Kupplungsvorsprung des anderen Kupplungselements einsetzbar ist, ein an dem ersten oder zweiten Kupplungselement angeordnetes Positionierungselement, welches in eine erste Vertiefung an dem anderen Kupplungselement einführbar ist, um beide Kupplungselemente in einer definierten Winkelposition um deren gemeinsame Achse zueinander zu positionieren, zumindest eine Lichtquelle, welche in einer zweiten Vertiefung an dem ersten oder zweiten Kupplungselement angeordnet ist und mit einem Lichtleiter an dem anderen Kupplungselement koppelbar ist, sowie zumindest ein an dem ersten oder zweiten Kupplungselement angeordnetes Übertragungselement, welches in eine dritte Vertiefung an dem anderen Kupplungselement einführbar ist, um Daten, Signale und/ oder Energie zwischen dem ersten und zweiten Kupplungselement sicher zu übertragen.

Gemäß einem ersten Ausführungsbeispiel der Kupplungsvorrichtung weist eines der beiden Elemente, das Positionierungselement oder die erste Vertiefung, eine Speichereinheit zur Speicherung von, vorzugsweise handstückbezogenen, Daten und das andere der beiden Elemente eine Leseeinheit auf, so dass Daten von der Speichereinheit des einen Kupplungselements mittels der Leseeinheit auf das andere Kupplungselement der Kupplungsvorrichtung übertragbar sind.

Zur leitungsgebundenen und/ oder drahtlosen Übertragung der Daten, Signale oder Energie von dem einen Kupplungselement zu dem anderen Kupplungselement umfasst hierzu das Positionierungselement und die erste Vertiefung sowie das Übertragungselement und die dritte Vertiefung bevorzugt elektrische Kontakte und/ oder Induktionsspulen. Die elektrischen Kontakte sind hierbei bevorzugt federnd gelagert, so dass diese vorzugsweise parallel zur Achse oder in radialer Richtung zur Achse der Kupplungsvorrichtung verschiebbar sind. Die drahtlose Übertragung von Daten und Signalen ist nicht auf Induktionsspulen beschränkt. Diese kann zum Beispiel auch optisch mittels infrarotem Licht erfolgen.

Gemäß einem zweiten Ausführungsbeispiel der Kupplungsvorrichtung weist eines der beiden Elemente, das Übertragungselement oder die dritte Vertiefung, zumindest einen Sensor auf, so dass Betriebsparameter des einen Kupplungselements, insbesondere des Handstücks oder der Antriebseinheit, mittels des Sensors erfassbar und auf das andere Kupplungselement der Kupplungsvorrichtung übertragbar sind. Der zumindest eine Sensor ist insbesondere dazu ausgebildet Werte wie Temperatur, Feuchtigkeit, Druck, Schall, oder Helligkeit in dem jeweils anderen Kupplungselement, insbesondere in dem Handstück oder der Antriebseinheit, zu erfassen.

Gemäß einem dritten Ausführungsbeispiel der Kupplungsvorrichtung weist eines der beiden Elemente, das Übertragungselement oder die dritte Vertiefung, eine Kamera auf, so dass Bilddaten, insbesondere eines zu bearbeitenden Gewebes, mittels der Kamera aufnehmbar und von dem einen Kupplungselement auf das andere Kupplungselement der Kupplungsvorrichtung übertragbar sind. Die Bilddaten lassen bevorzugt Rückschlüsse auf die stoffliche Beschaffenheit des zu bearbeitenden Gewebes zu.

Gemäß allen voranstehenden Ausführungsbeispielen ist das Positionierungselement und/ oder das Übertragungselement bevorzugt federnd gelagert, so dass dieses vorzugsweise parallel zur Achse der Kupplungsvorrichtung verschiebbar ist. Des Weiteren ist das Positionierungselement und/ oder das Übertragungselement vorzugsweise lösbar von dem Kupplungselement der Kupplungsvorrichtung ausgebildet. Hierzu weist das Kupplungselement bevorzugt eine Schraub-, Steck- und/ oder Schnappverbindung auf.

Um die Daten, Signale und/ oder Energie von dem einen Kupplungselement zu dem anderen Kupplungselement sicher zu übertragen, sind das Positionierungselement oder die erste Vertiefung, die zweite Vertiefung zur Aufnahme der Lichtquelle und das Übertragungselement oder die dritte Vertiefung an dem ersten oder zweiten Kupplungselement bevorzugt vor der Basisfläche, von der sich der Kupplungsvorsprung erstreckt, angeordnet.

Des Weiteren sind gemäß allen voranstehenden Ausführungsbeispielen das Positionierungselement oder die erste Vertiefung und die zweite Vertiefung für die Lichtquelle an dem ersten oder zweiten Kupplungselement in entgegengesetzter radialer Richtung zur gemeinsamen Achse der beiden Kupplungselemente angeordnet. Das Übertragungselement und die dritte Vertiefung sind bevorzugt um einen Winkel, von vorzugsweise 55 Grad, versetzt zur radialen Richtung der zweiten Vertiefung für die Lichtquelle an dem ersten und zweiten Kupplungselement positioniert.

Des Weiteren sind zur gemeinsamen Achse der beiden Kupplungselemente das Positionierungselement, das Übertragungselement sowie die zweite Vertiefung für die Lichtquelle an dem ersten und zweiten Kupplungselement vorzugsweise in einem gleichen radialen Abstand angeordnet.

Die vorliegende Kupplungsvorrichtung zeichnet sich durch folgende Vorteile aus.

Die erfindungsgemäße Kupplungsvorrichtung ermöglicht Signale, Daten und/ oder Energie von dem einen Kupplungselement auf das andere Kupplungselement der Kupplungsvorrichtung sicher zu übertragen. Durch die beabstandete Anordnung des Positionierungselements und des Übertragungselements, insbesondere der elektrischen Kontakte und der Induktionsspulen zur drahtgebundenen und drahtlosen Energie- und Datenübertragung, an dem ersten und zweiten Kupplungselement der Kupplungsvorrichtung wird die Erzeugung von Gegenfeldern bei der Energie- und Datenübertragung vermieden. Des Weiteren ist eine Abschirmung der elektrischen Kontakte und Induktionsspulen nicht notwendig.

Einen weiteren Vorteil der Erfindung bildet die Anordnung der elektrischen Kontakte, der Induktionsspulen, der Sensoren sowie der Kamera zur Übertragung von Signalen, Daten und/ oder Energie außerhalb der metallischen Bauteile der Kupplungsvorrichtung, insbesondere außerhalb der Hülse für die Kupplungsausnehmung und außerhalb des Kupplungsvorsprungs, in dem Übertragungselement und/ oder in dem Positionierungselement der Kupplungsvorrichtung. Hierdurch sind diese Komponenten von mehreren Seiten her zugänglich und in das jeweils andere Kupplungselement, insbesondere in das Handstück, der Antriebseinheit oder in den Versorgungsschlauch, einführbar, so dass insbesondere Signale und Daten direkt in dem jeweils anderen Kupplungselement erfassbar sind.

Des Weiteren ermöglicht die Kupplungsvorrichtung Signale, Daten und/ oder Energie für mehrere elektronische Komponenten in dem Handstück, der Antriebseinheit und/ oder dem Versorgungsschlauch gleichzeitig bzw. parallel von dem einen Kupplungselement auf das andere Kupplungselement der Kupplungsvorrichtung sicher zu übertragen.

Einen weiteren Vorteil der Erfindung bildet die Kompatibilität der Kupplungsvorrichtung, insbesondere der Kupplungselemente, mit bereits bestehenden Kupplungselementen, insbesondere mit bereits bestehenden zahnärztlichen Handstücken. Dadurch, dass das Übertragungselement zum Senden und/ oder Empfangen von Signalen, Daten und/ oder Energie bevorzugt an dem Kupplungselement des Handstücks angeordnet ist, ist es möglich bestehende Handstücke mit dem jeweils anderen Kupplungselement der erfindungsgemäßen Kupplungsvorrichtung zu koppeln. Die Antriebseinheiten sind somit weiterhin ohne funktionelle Einschränkung kompatibel mit den bestehenden medizinischen Handstücken bzw. mit deren Kupplungselementen.

Im Rahmen der Erfindung versteht es sich selbstverständlich, dass die oben beschriebene Kupplungsvorrichtung nicht auf die Verwendung bei einem medizinischen, insbesondere zahnärztlichen, Handstück und einer Antriebseinheit beschränkt ist. Die Kupplungsvorrichtung kann vielmehr zur lösbaren Verbindung zweier medizinischer, insbesondere zahnärztlicher, Vorrichtungen verwendet werden. Vorzugsweise ist zumindest eine der medizinischen, insbesondere zahnärztlichen, Vorrichtungen als Reinigungs- und/ oder Pflegegerät, als Antriebseinheit, Versorgungsschlauch, Diagnosegerät oder als Handstück mit einer Antriebsvorrichtung für ein Werkzeug ausgebildet.

Nachfolgend wird die Erfindung anhand mehrerer Ausführungsbeispiele und in Verbindung mit den beigefügten Zeichnungen erläutert.

Dabei zeigt / zeigen die:
Figur 1 ein erstes Ausführungsbeispiel der Kupplungsvorrichtung mit einem ersten Kupplungselement an einer Antriebseinheit und einem zweiten Kupplungselement an einem medizinischen, insbesondere zahnärztlichen, Handstück,
Figur 2 ein zweites Ausführungsbeispiel der Kupplungsvorrichtung zur Übertragung von Signalen, Daten und/ oder Energie, einer Antriebsbewegung und eines Arbeitsmediums zwischen dem Handstück und der Antriebseinheit,
Figur 3 ein drittes Ausführungsbeispiel der Kupplungsvorrichtung,
Figuren 4A bis 4C die Ausführungsbeispiele des ersten Kupplungselements der Kupplungsvorrichtung aus den Figuren 1 bis 3 in Draufsicht.

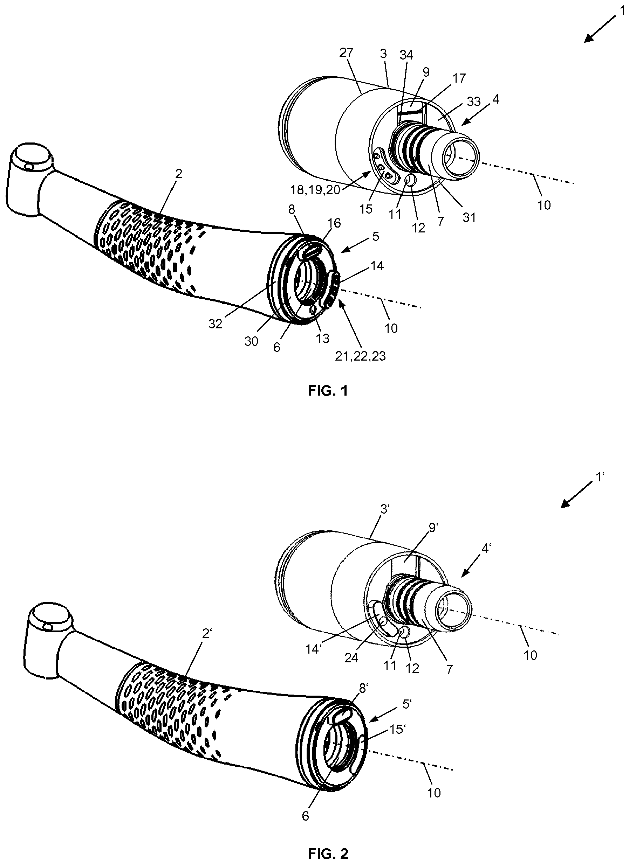

In Figur 1 ist ein erstes Ausführungsbeispiel der Kupplungsvorrichtung 1 mit einem ersten Kupplungselement 4 an einer Antriebseinheit 3 und einem zweiten Kupplungselement 5 an einem medizinischen, insbesondere zahnärztlichen, Handstück 2 gezeigt. Zur lösbaren Verbindung des Handstücks 2 mit der Antriebseinheit 3 und zur Übertragung einer Antriebsbewegung sowie eines Arbeitsmediums, insbesondere eines Fluids, weist das erste Kupplungselement 4 einen Kupplungsvorsprung 7 auf, welcher in eine Kupplungsausnehmung 6 an dem zweiten Kupplungselement 5 bis zu einer Anschlagfläche 34 einführbar ist. Die Anschlagfläche 34 ist hierbei bevorzugt an dem Kupplungsvorsprung 7 angeordnet. Die Länge des Kupplungsvorsprungs 7, gemessen von der Anschlagfläche 34, beträgt vorzugsweise weniger als 23 Millimeter, insbesondere 21,60 Millimeter, um in der mindestens 23 Millimeter tiefen Kupplungsausnehmung 6 aufnehmbar zu sein. Um eine in einer Vertiefung 12 angeordnete Lichtquelle 11, insbesondere eine Leuchtdiode, der Antriebseinheit 3 zu einem korrespondierenden Lichtleiter 13 an dem medizinischen Handstück 2 auszurichten, weist die Kupplungsvorrichtung 1, insbesondere das zweite Kupplungselement 5 mit der Kupplungsausnehmung 6, ein Positionierungselement 8 auf, welches in eine erste Vertiefung 9 an dem ersten Kupplungselement 4 einführbar ist. Hierdurch können beide Kupplungselemente 4, 5 in einer definierten Winkelposition um deren gemeinsame Achse 10 zueinander positioniert werden. Das Positionierungselement 8 erstreckt sich bevorzugt an dem zweiten Kupplungselement 5 in entgegengesetzter Richtung zur Kupplungsausnehmung 6 und ist insbesondere als Nase, Fortsatz oder Vorsprung ausgebildet. Des Weiteren ist das Positionierungselement 8 bevorzugt federnd gelagert, so dass dieses vorzugsweise parallel zur Drehachse 10 der Kupplungsvorrichtung 1 verschiebbar ist.

Zur automatischen Erkennung des an die Antriebseinheit 3 anschließbaren medizinischen Handstücks 2, weist das Positionierungselement 8 bevorzugt eine Speichereinheit 16 zur Speicherung von, vorzugsweise handstückbezogenen, Daten auf. Mittels einer Leseeinheit 17 in der ersten Vertiefung 9 können diese Daten von der Speichereinheit 16 ausgelesen und auf das Kupplungselement 4 der Antriebseinheit 3 übertragen werden. Die Übertragung erfolgt bevorzugt mittels zweier Induktionsspulen, wobei jeweils eine Spule in dem Positionierungselement 8 und eine Spule in der Vertiefung 9 angeordnet ist. Daten können so auch von der Antriebseinheit 3 auf die Speichereinheit 16 des Handstücks 2 übertragen werden. Die Speichereinheit 16 sowie die dazugehörige Induktionsspule sind bevorzugt außerhalb des Handstücks 2 in dem Positionierungselement 8 angeordnet, so dass diese von mehreren Seiten für das andere Kupplungselement 4, insbesondere für die Leseeinheit 17 frei zugänglich sind. Des Weiteren ist das Positionierungselement 8 mit der Speichereinheit 16 bevorzugt lösbar von dem Kupplungselement 5 ausgebildet ist, so dass dieses austauschbar ist.

Die Leseeinheit 17 ist in diesem Ausführungsbeispiel bevorzugt vor einer Basisfläche 27 von der sich der Kupplungsvorsprung 7 erstreckt angeordnet. Hierdurch wird eine sichere Datenübertragung zwischen der Speichereinheit 16 und der Leseeinheit 17 gewährleistet. Elektrisch leitende Bauteile, welche die Datenübertragung auf Grund von elektrischen oder magnetischen Gegenfeldern gefährden, wie zum Beispiel die Basisfläche 27 des Kupplungsvorsprungs, sind nicht zwischen der Speichereinheit 16 und der Leseeinheit 17 angeordnet.

Zur Übertragung von Signalen, Daten und/ oder Energie zwischen den beiden Kupplungselementen 4, 5 umfasst das zweite Kupplungselement 5 ein Übertragungselement 14, welches in eine dritte Vertiefung 15 an dem ersten Kupplungselement 4 einführbar ist. Das Übertragungselement 14 erstreckt sich, wie das Positionierungselement 8, bevorzugt von dem zweiten Kupplungselement 5, insbesondere von einer ringförmigen Stirnfläche 30, parallel zur Achse 10 in entgegengesetzter Richtung zur Kupplungsausnehmung 6. Des Weiteren erstreckt sich das Übertragungselement 14 bevorzugt in radialer Richtung um die Achse 10 der Kupplungsvorrichtung 1 und weist dadurch einen kreissegmentförmigen Querschnitt auf. Wie das Positionierungselement 8 kann auch das Übertragungselement 14 federnd, insbesondere verschiebbar und lösbar, von dem zweiten Kupplungselement 5 ausgebildet sein. Der Überstand beider Elemente 8, 14 von der Anschlagfläche 34 beträgt insbesondere zwischen 1,50 und 2,30 Millimeter, bevorzugt 2,25 Millimeter, um in die Vertiefungen 9, 15, welche mindestens 2,50 Millimeter tief ausgebildet sind, einführbar zu sein.

Zur leitungsgebundenen Übertragung der Daten, Signale und/ oder der Energie umfasst das Übertragungselement 14, welches ebenfalls die Form einer Nase, eines Fortsatzes oder eines Vorsprungs aufweist, sowie die dritte Vertiefung 15 bevorzugt elektrische Kontakte 18, 19, 20; 21, 22, 23. In diesem ersten Ausführungsbeispiel weist das Übertragungselement 14 sowie die Vertiefung jeweils drei elektrische Kontakte 18, 19, 20; 21, 22, 23 auf. Jeweils zwei der elektrischen Kontakte 18, 19 und 21, 22 dienen hierbei bevorzugt zur Übertragung von elektrischer Energie. Die weiteren elektrischen Kontakte 20, 23 dienen vorzugsweise zur Übertragung von elektrischen Signalen, insbesondere von Wurzelkanalpositionssignalen. Die elektrischen Kontakte 18, 19, 20; 21, 22, 23 sind bevorzugt federnd gelagert, so dass diese vorzugsweise in radialer Richtung zur Achse 10 der Kupplungsvorrichtung 1 verschiebbar sind. Insbesondere sind die elektrischen Kontakte 18, 19, 20 des ersten Kupplungselements 4 als Kontaktstifte ausgebildet, welche in elektrische Kontakte 21, 22, 23 mit in radialer Richtung federnd gelagerten Laschen des zweiten Kupplungselements 5 einführbar sind. Alternativ könne die elektrischen Kontakte 18, 19, 20; 21, 22, 23 auch parallel zur Achse 10 verschiebbar gelagert sein.

Um schließlich die Signal, Daten und/ oder Energieübertragung sowie die Lichtübertragung zwischen dem ersten Kupplungselement 4 und dem zweiten Kupplungselement 5 vor äußeren Einflüssen zu schützen umfasst eines der beiden Kupplungselemente 4, 5, insbesondere das erste Kupplungselement 4, einen ringförmigen Vorsprung 31. Der Vorsprung 31 erstreckt sich hierbei in axialer Richtung von einer ringförmigen Stirnfläche 33 des ersten Kupplungselements 4. In einem gekuppelten Zustand beider Kupplungselemente 4, 5 umgibt so der ringförmige Vorsprung 31 zumindest teilweise den Kupplungsvorsprung 7, die Kupplungsausnehmung 6, das Positionierungselement 8, die Lichtquelle 11, das Übertragungselement 14 sowie eine äußere Mantelfläche 32 des zweiten Kupplungselements 5.

Figur 2 zeigt ein zweites Ausführungsbeispiel der Kupplungsvorrichtung 1' mit einem Kupplungsvorsprung 7 an einem ersten Kupplungselement 4' und einer Kupplungsausnehmung 6 an einem zweiten Kupplungselement 5'. Hierbei ist das Übertragungselement 14' an dem ersten Kupplungselement 4' der Antriebseinheit 3' und die dritte Vertiefung 15' an dem zweiten Kupplungselement 5' des Handstücks 2' angeordnet. Das Übertragungselement 14' erstreckt sich parallel zum Kupplungsvorsprung 7. Um wieder beide Kupplungselemente 4', 5' in einer definierten Winkelposition um deren gemeinsame Achse 10 zueinander positioniert, weist das Handstück 2' ein Positionierungselement 8' auf, welches in eine Vertiefung 9' an der Antriebseinheit 3' einführbar ist. Die zweite Vertiefung 12 zur Aufnahme der Lichtquelle 11 ist auch in diesem Ausführungsbeispiel in entgegengesetzter radialer Richtung von der gemeinsamen Achse 10 zur Vertiefung 9' für das Positionierungselement 8' an der Antriebseinheit 3' angeordnet.

Um Betriebsparameter des zweiten Kupplungselements 5', insbesondere des Handstücks 2', zu erfassen und auf das erste Kupplungselement 4' der Kupplungsvorrichtung 1', insbesondere auf die Antriebseinheit 3' zu übertragen, umfasst in diesem Ausführungsbeispiel das Übertragungselement 14' zumindest einen Sensor 24. Der Sensor 24 ist bevorzugt außerhalb der Antriebseinheit 3' in dem Übertragungselement 14' angeordnet, so dass dieser für das Handstück 2' von mehreren Seiten her frei zugänglich ist. Der Sensor 24 ist vorzugsweise ausgebildet Temperaturen, Feuchtigkeit, Druck, Schall, oder Helligkeit in dem zweiten Kupplungselement 5', insbesondere in dem Handstück 2', zu erfassen. Des Weiteren ist der zumindest eine Sensor 24 insbesondere dazu ausgebildet die gemessenen Werte drahtlos oder leitungsgebunden an eine Steuerung der Antriebseinheit 3' zu übertragen. Abhängig von den erfassten Betriebsparametern können Arbeitsmedien, wie zum Beispiel Licht, Wasser, Luft oder Spray zur Kühlung des Handstücks 2', automatisch dem über einen Versorgungsschlauch mit einer zahnärztlichen Einheit verbundenen medizinischen Handstück 2' zugeführt werden.

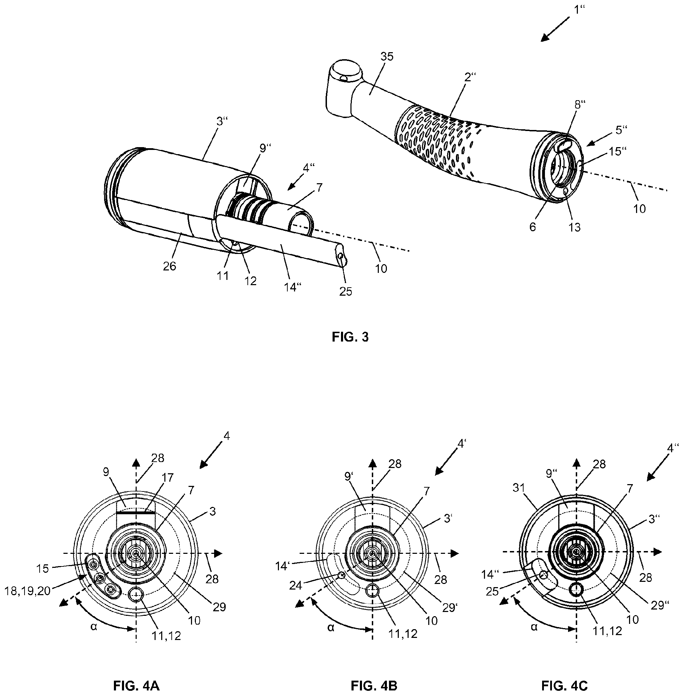

In Figur 3 ist ein drittes Ausführungsbeispiel der Kupplungsvorrichtung 1" mit einem ersten und zweiten Kupplungselement 4", 5" gezeigt. Das erste Kupplungselement 4" an der Antriebseinheit 3" weist, wie in den Ausführungsbeispielen zuvor, einen Kupplungsvorsprung 7 auf, welcher in eine Kupplungsausnehmung 6 an dem zweiten Kupplungselement 5" des medizinischen Handstücks 2" einführbar ist. Auch das Positionierungselement 8" sowie die Vertiefung 15" zur Aufnahme eines Übertragungselements 14" sind in diesem Ausführungsbeispiel, wie in Figur 2, an dem medizinischen Handstück 2" angeordnet. Zusätzlich zu dem Positionierungselement 8" sowie der Vertiefung 15" weist das zweite Kupplungselement 5" eine Ausnehmung für einen Lichtleiter 13 auf. Die erste Vertiefung 9" zur Aufnahme des Positionierungselements 8", die zweite Vertiefung 12 für die Lichtquelle 11 sowie das Übertragungselement 14" sind an der Antriebseinheit 3" vorgesehen.

Um Bilddaten, insbesondere eines zu bearbeitenden Gewebes, aufzunehmen und von dem zweiten Kupplungselement 5" des medizinischen Handstücks 2" auf das erste Kupplungselement 4" der Antriebseinheit 3" zu übertragen, umfasst das Übertragungselement 14" der ersten Kupplungsvorrichtung 4" eine Kamera 25. Die Kamera 25 ist hierbei an einer vorderen Stirnfläche des Übertragungselements 14" positioniert. Damit die Bilddaten direkt im Bereich des mit dem medizinischen Handstücks 2" koppelbaren Behandlungswerkzeugs erfasst werden können, erstreckt sich die Vertiefung 15" von dem Kupplungselement 5" bevorzugt bis in einen Halsbereich 35 des Handstücks 2", welcher ein Kopfstück mit einer Werkzeugaufnahme mit dem Griffstück des Handstücks 2" verbindet. Die Vertiefung 15" ist hierzu bevorzugt durch ein Führungsrohr gebildet. An dem kopfseitigen Ende des medizinischen Handstücks 2" weist dieses bevorzugt eine Optik auf, welche Bilddaten von dem zu behandelnden Gewebe auf die Kamera 25 lenkt. Alternativ kann auch ein bildgebender Leiter in der Vertiefung 15" angeordnet sein.

Bevorzugt ist das Übertragungselement 14" mit der Kamera 25 lösbar von dem Kupplungselement 4", insbesondere lösbar von der Antriebseinheit 3", ausgebildet. Hierzu weist die Kupplungsvorrichtung 1" vorzugsweise eine Steck- bzw. Schnappverbindung 26 auf, welche durch eine Nut gebildet ist, die sich in axialer Richtung von einem ersten Ende der Antriebseinheit 3" bis zu dem ersten Kupplungselement 4" erstreckt. Des Weiteren erstreckt sich das Übertragungselement 14" bevorzugt parallel zur Achse 10 und zum Kupplungsvorsprung 7.

Erfasste Bilddaten sind von der Kamera 25 bevorzugt leitungsgebunden an eine Steuerung der Antriebseinheit 3", insbesondere an eine zahnärztliche Einheit mit einer Anzeige, übertragbar. Hierzu weist das Übertragungselement 14" bevorzugt mehrere elektrische Leitungen auf, welche sich von der Kamera 25 bis zu einem rückseitigen Ende des Übertragungselements 14" oder direkt bis zu der zahnärztlichen Einheit erstrecken.

Die Figuren 4A bis 4C zeigen die mehreren Ausführungsbeispiele des ersten Kupplungselements 4, 4', 4" der Kupplungsvorrichtung 1, 1', 1" aus den Figuren 1 bis 3 in der Draufsicht.

Dabei zeigt die Figur 4A das erste Ausführungsbeispiel des ersten Kupplungselements 4 mit dem Kupplungsvorsprung 7, welches an der Antriebseinheit 3 angeordnet ist. Die Vertiefung 12 für die Lichtquelle 11 sowie die Vertiefung 15 mit den elektrischen Kontakten 18, 19, 20 zur Aufnahme des Übertragungselements sind hierbei bevorzugt in einem gleichen radialen Abstand 29 zur gemeinsamen Achse 10 an dem Kupplungselement 4 angeordnet. Des Weiteren ist die erste Vertiefung 9 für das Positionierungselement mit der Leseeinheit 17 und die zweite Vertiefung 12 für die Lichtquelle 11 an dem Kupplungselement 4 in entgegengesetzter radialer Richtung 28 zur gemeinsamen Achse 10 angeordnet. Die Vertiefung 15 für das Übertragungselement ist um einen Winkel α, von vorzugsweise 55 Grad, versetzt zur radialen Richtung der zweiten Vertiefung 12 für die Lichtquelle 11 an dem Kupplungselement 4 positioniert.

In Figur 4B ist das zweite Ausführungsbeispiel des ersten Kupplungselements 4' mit dem Kupplungsvorsprung 7 an der Antriebseinheit 3' gezeigt. Auch in diesem Ausführungsbeispiel sind die Vertiefung 12 für die Lichtquelle 11 sowie das Übertragungselement 14' mit dem zumindest einen Sensor 24 in einem gleichen radialen Abstand 29' zur gemeinsamen Achse 10 an dem Kupplungselement 4' angeordnet. Der Abstand beträgt vorzugsweise 7,50 Millimeter. In radialer Richtung 28 zur gemeinsamen Achse 10 sind die erste Vertiefung 9' für das Positionierungselement und die zweite Vertiefung 12 für die Lichtquelle 11 an dem Kupplungselement 4' ebenso in entgegengesetzter Richtung positioniert. Das Übertragungselement 14', welches um den Winkel α versetzt zur radialen Richtung 28 der zweiten Vertiefung 12 an dem Kupplungselement 4' angeordnet ist, erstreckt sich hierbei bevorzugt um einen Winkel von 70 Grad und weist dadurch einen kreissegmentförmigen Querschnitt auf.

In dem in Figur 4C gezeigten Ausführungsbeispiel des ersten Kupplungselements 4" mit dem Kupplungsvorsprung 7 an der Antriebseinheit 3" weist das Übertragungselement 14" eine Kamera 25 auf. Die Kamera 25 und das Übertragungselement 14" sind, wie in den vorherigen Ausführungsbeispielen, um einen Winkel a, von vorzugsweise 55 Grad, versetzt zur radialen Richtung 28 der zweiten Vertiefung 12 für die Lichtquelle 11 und in einem gleichen radialen Abstand 29" zur gemeinsamen Achse 10 wie die Vertiefung 12 an dem Kupplungselement 4" positioniert. Die Vertiefung 9" für das Positionierungselement erstreckt sich hierbei in radialer Richtung 28 von dem Kupplungszapfen 7 bis zu dem ringförmigen Vorsprung 31.

Die Erfindung ist nicht auf die beschriebenen Ausführungsbeispiele beschränkt, sondern ist durch die Patentansprüche definiert.

## Patentansprüche

1. Kupplungsvorrichtung (1, 1', 1") zur lösbaren Verbindung eines medizinischen, insbesondere zahnärztlichen, Handstücks (2, 2', 2") mit einer Antriebseinheit (3, 3', 3") oder einem Versorgungsschlauch zur Übertragung von Signalen, Daten und/ oder Energie, einer Antriebsbewegung, eines Arbeitsmediums und/ oder von Licht zwischen dem Handstück (2, 2', 2") und der Antriebseinheit (3, 3', 3") oder dem Versorgungsschlauch, wobei die Kupplungsvorrichtung (1, 1', 1") sich entlang einer Längsachse (10) erstreckt und umfasst: ein erstes und zweites Kupplungselement (4, 4', 4"; 5, 5', 5"), wobei eines der beiden Kupplungselemente (5, 5', 5") als Kupplungsausnehmung (6) ausgebildet ist, in die ein Kupplungsvorsprung (7) des anderen Kupplungselements (4, 4', 4") einsetzbar ist, wobei die Kupplungsausnehmung (6) und der Kupplungsvorsprung (7) jeweils von einer ringförmigen Stirnfläche (30, 33) umgeben sind, ein an der ringförmigen Stirnfläche (30, 33) des ersten oder zweiten Kupplungselements (5, 5', 5") angeordnetes Positionierungselement (8, 8', 8"), welches in eine erste Vertiefung (9, 9', 9") an der ringförmigen Stirnfläche (30, 33) des anderen Kupplungselements (4, 4', 4") einführbar ist, um beide Kupplungselemente (4, 4', 4"; 5, 5', 5") in einer definierten Winkelposition um deren gemeinsame Achse (10) zueinander zu positionieren, zumindest eine Lichtquelle (11), welche in einer zweiten Vertiefung (12) an der ringförmigen Stirnfläche (30, 33) des ersten oder zweiten Kupplungselement (4, 4', 4") angeordnet ist und mit einem Lichtleiter (13) an der ringförmigen Stirnfläche (30, 33) des anderen Kupplungselements (5, 5") koppelbar ist, sowie zumindest ein an der ringförmigen Stirnfläche (30, 33) des ersten oder zweiten Kupplungselements (4', 4"; 5) angeordnetes Übertragungselement (14, 14', 14"), welches in eine dritte Vertiefung (15, 15', 15") an der ringförmigen Stirnfläche (30, 33) des anderen Kupplungselements (4; 5', 5") einführbar ist, um Daten, Signale und/ oder Energie zwischen dem ersten und zweiten Kupplungselement (4, 4', 4"; 5, 5', 5") zu übertragen, **dadurch gekennzeichnet, dass**
in einer Draufsicht auf die ringförmigen Stirnflächen (30, 33) des ersten und zweiten Kupplungselements (4, 4', 4"; 5, 5', 5") das Übertragungselement (14, 14', 14") und die dritte Vertiefung (15, 15', 15")
(i) durch einen Abschnitt der ringförmigen Stirnflächen (30, 33) von dem Positionierungselement (8, 8', 8") und der ersten Vertiefung (9, 9', 9") beabstandet und
(ii) der ringförmigen Stirnfläche (30, 33) um die Kupplungsausnehmung (6) oder den Kupplungsvorsprung (7) entlang einem radialen Abstand (29, 29', 29") zur Längsachse (10) folgend zwischen dem Positionierungselement (8, 8', 8") oder der ersten Vertiefung (9, 9', 9") und der Lichtquelle (11) oder der zweiten Vertiefung (12) oder dem Lichtleiter (13)
angeordnet sind.

2. Kupplungsvorrichtung (1) nach Anspruch 1, **dadurch gekennzeichnet, dass**
das Positionierungselement (8) oder die erste Vertiefung (9) eine Speichereinheit (16) zur Speicherung von, vorzugsweise handstückbezogenen, Daten und das andere dieser beiden Elemente, nämlich das Positionierungselement (8) oder die erste Vertiefung (9), eine Leseeinheit (17) aufweist, so dass Daten von der Speichereinheit (16) des einen Kupplungselements (5) mittels der Leseeinheit (17) auf das andere Kupplungselement (4) der Kupplungsvorrichtung (1) übertragbar sind.

3. Kupplungsvorrichtung (1) nach Anspruch 1 oder 2, **dadurch gekennzeichnet, dass**
das Positionierungselement (8) und die erste Vertiefung (9) und/ oder das Übertragungselement (14) und die dritte Vertiefung (15) elektrische Kontakte (18, 19, 20; 21, 22, 23) und/ oder Induktionsspulen zur leitungsgebundenen und/ oder drahtlosen Übertragung von Daten, Signalen oder Energie von dem einen Kupplungselement (4) zu dem anderen Kupplungselement (5) aufweisen.

4. Kupplungsvorrichtung (1) nach Anspruch 3, **dadurch gekennzeichnet, dass**
die elektrischen Kontakte (18, 19, 20; 21, 22, 23) federnd gelagert sind, so dass diese vorzugsweise parallel zur Achse (10) oder in radialer Richtung (28) zur Achse (10) der Kupplungsvorrichtung (1) verschiebbar sind.

5. Kupplungsvorrichtung (1') nach einem der vorherigen Ansprüche, **dadurch gekennzeichnet, dass**
das Übertragungselement (14') oder die dritte Vertiefung (15') zumindest einen Sensor (24) aufweist, so dass Betriebsparameter des einen Kupplungselements (5'), insbesondere des Handstücks (2') oder der Antriebseinheit (3'), mittels des Sensors (24) erfassbar und auf das andere Kupplungselement (4') der Kupplungsvorrichtung (1') übertragbar sind.

6. Kupplungsvorrichtung (1") nach einem der vorherigen Ansprüche, **dadurch gekennzeichnet, dass**
das Übertragungselement (14") oder die dritte Vertiefung (15"), eine Kamera (25) aufweist, so dass Bilddaten, insbesondere eines zu bearbeitenden Gewebes, mittels der Kamera (25) aufnehmbar und von dem einen Kupplungselement (5") auf das andere Kupplungselement (4") der Kupplungsvorrichtung (1") übertragbar sind.

7. Kupplungsvorrichtung (1, 1', 1") nach einem der vorherigen Ansprüche, **dadurch gekennzeichnet, dass**
das Positionierungselement (8, 8', 8") und/ oder das Übertragungselement (14, 14', 14") federnd gelagert ist/sind, so dass dieses/diese vorzugsweise parallel zur Achse (10) der Kupplungsvorrichtung (1, 1', 1") verschiebbar ist/sind.

8. Kupplungsvorrichtung (1, 1', 1") nach einem der vorherigen Ansprüche, **dadurch gekennzeichnet, dass**
das Positionierungselement (8, 8', 8") und/ oder das Übertragungselement (14, 14', 14") lösbar von dem ersten oder zweiten Kupplungselement (4', 4"; 5, 5', 5") der Kupplungsvorrichtung (1, 1', 1") ausgebildet ist/sind.

9. Kupplungsvorrichtung (1") nach Anspruch 8, **dadurch gekennzeichnet, dass**
das Kupplungselement (4") zur lösbaren Aufnahme des Positionierungselements (8") und/ oder des Übertragungselements (14") eine Schraub-, Steck- und/ oder Schnappverbindung (26) aufweist.

10. Kupplungsvorrichtung (1, 1', 1") nach einem der vorherigen Ansprüche, **dadurch gekennzeichnet, dass**
das Positionierungselement (8, 8', 8") oder die erste Vertiefung (9, 9', 9"), die zweite Vertiefung (12) zur Aufnahme der Lichtquelle (11) und das Übertragungselement (14, 14', 14") oder die dritte Vertiefung (15, 15', 15") an dem ersten oder zweiten Kupplungselement (4, 4', 4"; 5, 5', 5") beabstandet von einer Basisfläche (27), von der sich der Kupplungsvorsprung erstreckt, angeordnet sind, um Daten, Signale und/ oder Energie von dem einen Kupplungselement (4, 4', 4') zu dem anderen Kupplungselement (5, 5', 5") sicher zu übertragen.

11. Kupplungsvorrichtung (1, 1', 1") nach einem der vorherigen Ansprüche, **dadurch gekennzeichnet, dass**
das Positionierungselement (8, 8', 8") oder die erste Vertiefung (9, 9', 9") und die zweite Vertiefung (12) für die Lichtquelle (11) an dem ersten oder zweiten Kupplungselement (4, 4', 4") in entgegengesetzter radialer Richtung (28) zur gemeinsamen Achse (10) der beiden Kupplungselemente (4, 4', 4"; 5, 5', 5") angeordnet sind.

12. Kupplungsvorrichtung (1, 1', 1") nach einem der vorherigen Ansprüche, **dadurch gekennzeichnet, dass**
das Übertragungselement (14, 14', 14") und die dritte Vertiefung (15, 15', 15") um einen Winkel (a), von vorzugsweise 55 Grad, versetzt zur radialen Richtung (28) der zweiten Vertiefung (12) für die Lichtquelle (11) an dem ersten und zweiten Kupplungselement (4, 4', 4"; 5, 5', 5") angeordnet sind.

13. Kupplungsvorrichtung (1, 1', 1") nach einem der vorherigen Ansprüche, **dadurch gekennzeichnet, dass**
das Positionierungselement (8, 8', 8"), das Übertragungselement (14, 14', 14") sowie die zweite Vertiefung (12) für die Lichtquelle (11) an dem ersten und zweiten Kupplungselement (4, 4', 4"; 5, 5', 5") in einem gleichen radialen Abstand (29, 29', 29") zur gemeinsamen Achse (10) der beiden Kupplungselemente (4, 4', 4"; 5, 5', 5") angeordnet sind.

14. Verwendung einer Kupplungsvorrichtung (1, 1', 1") nach einem der vorherigen Ansprüche zur lösbaren Verbindung zweier medizinischer, insbesondere zahnärztlicher, Vorrichtungen, wobei vorzugsweise zumindest eine dieser zwei medizinischen, insbesondere zahnärztlichen, Vorrichtungen als Reinigungs- und/ oder Pflegegerät, als Antriebseinheit (3, 3', 3"), Versorgungsschlauch, Diagnosegerät oder als Handstück (2, 2', 2") ausgebildet ist.

15. Medizinische, insbesondere zahnärztliche, Vorrichtung, umfassend ein Handstück (2, 2', 2") und eine Antriebseinheit (3, 3', 3") oder einen Versorgungsschlauch, **gekennzeichnet durch**
eine Kupplungsvorrichtung (1, 1', 1") nach einem der Ansprüche 1 - 13 zur lösbaren Verbindung des Handstücks (2, 2', 2") mit der Antriebseinheit (3, 3', 3") oder dem Versorgungsschlauch.

## Claims

1. A coupling device (1, 1', 1") for releasable connection of a medical, in particular dental, handpiece (2, 2', 2") to a drive unit (3, 3', 3") or to a supply tubing for transferring signals, data and/or energy, a drive movement, a working medium and/or light between the handpiece (2, 2', 2") and the drive unit (3, 3', 3") or the supply tubing, wherein the coupling device (1, 1', 1") extends along a longitudinal axis (10) and comprises: a first and second coupling element (4, 4', 4"; 5, 5', 5"), wherein one of the two coupling elements (5, 5', 5") is designed as a coupling cavity (6) into which a coupling protrusion (7) of the other coupling element (4, 4', 4") can be inserted, wherein the coupling cavity (6) and the coupling protrusion (7) each are surrounded by an annular end face (30, 33), a positioning element (8, 8', 8") which is disposed on the annular end face (30, 33) of the first or second coupling element (5, 5', 5") and can be inserted into a first recess (9, 9', 9") on the annular end face (30, 33) of the other coupling element (4, 4', 4") in order to position the two coupling elements (4, 4', 4"; 5, 5', 5") in a defined angular position about their common axis (10) relative to one another, at least one light source (11) which is disposed in a second recess (12) on the annular end face (30, 33) of the first or second coupling element (4, 4', 4") and is couplable to an optical fiber (13) on the annular end face (30, 33) of the other coupling element (5, 5"), and at least one transfer element (14, 14', 14"), which is disposed on the annular end face (30, 33) of the first or second coupling element (4', 4"; 5) and can be inserted into a third recess (15, 15', 15") on the annular end face (30, 33) of the other coupling element (4; 5', 5") to transfer data, signals and/or energy between the first and second coupling elements (4, 4', 4"; 5, 5', 5"), **characterized in that**
in a top-view of the annular end faces (30, 33) of the first and second coupling element (4, 4', 4"; 5, 5', 5") the transfer element (14, 14', 14") and the third recess (15, 15', 15")
(i) are separated by a segment of the annular end faces (30, 33) from the positioning element (8, 8', 8") and the first recess (9, 9', 9") and
(ii) are disposed between the positioning element (8, 8', 8") or the first recess (9, 9', 9") and the light source (11) or the second recess (12) or the optical fiber (13) when following the annular end face (30, 33) around the coupling cavity (6) or the coupling protrusion (7) along a radial distance (29, 29', 29") from the longitudinal axis (10).

2. The coupling device (1) according to claim 1, **characterized in that** the positioning element (8) or the first recess (9) comprises a memory unit (16) for storing data, preferably handpiece-related data, and the other one of the two elements, namely the positioning element (8) or the first recess (9), comprises a reader unit (17), so that data can be transferred from the memory unit (16) of the one coupling element (5) to the other coupling element (4) of the coupling device (1) by the reader unit (17).

3. The coupling device (1) according to claim 1 or 2, **characterized in that** the positioning element (8) and the first recess (9) and/or the transfer element (14) and the third recess (15) comprise electric contacts (18, 19, 20; 21, 22, 23) and/or induction coils for hardwired and/or wireless transfer of data, signals or energy from the one coupling element (4) to the other coupling element (5).

4. The coupling device (1) according to claim 3, **characterized in that** the electric contacts (18, 19, 20; 21, 22, 23) are spring-mounted, so that they are displaceable, preferably in parallel with the axis (10) or in the radial direction (28) to the axis (10) of the coupling device (1).

5. The coupling device (1') according to any one of the preceding claims, **characterized in that**
the transfer element (14') or the third recess (15') comprises at least one sensor (24), so that operating parameters of the one coupling element (5'), in particular of the handpiece (2') or of the drive unit (3') can be detected by means of the sensor (24) and can be transferred to the other coupling element (4') of the coupling device (1').

6. The coupling device (1") according to any one of the preceding claims, **characterized in that**
the transfer element (14") or the third recess (15") comprises a camera (25) so that image data, in particular of tissue to be treated, can be recorded by the camera (25) and can be transferred from the one coupling element (5") to the other coupling element (4") of the coupling device (1").

7. The coupling device (1, 1', 1") according to any one of the preceding claims, **characterized in that**
the positioning element (8, 8', 8") and/or the transfer element (14, 14', 14") is/are spring-mounted, so that this is/they are displaceable preferably in parallel with the axis (10) of the coupling device (1, 1', 1").

8. The coupling device (1, 1', 1") according to any one of the preceding claims, **characterized in that**
the positioning element (8, 8', 8") and/or the transfer element (14, 14', 14") is/are designed to be releasable from the first or second coupling element (4', 4"; 5, 5', 5") of the coupling device (1, 1', 1").

9. The coupling device (1") according to claim 8, **characterized in that**
the coupling element (4") comprises a screw connection, a plug connection and/or a snap connection (26) for releasable accommodation of the positioning element (8") and/or of the transfer element (14").

10. The coupling device (1, 1', 1") according to any one of the preceding claims, **characterized in that**
the positioning element (8, 8', 8") or the first recess (9, 9', 9"), the second recess (12) to accommodate the light source (11) and the transfer element (14, 14', 14") or the third recess (15, 15', 15") are disposed on the first or second coupling element (4, 4', 4"; 5, 5', 5") and spaced from a base surface (27), from which the coupling protrusion extends, in order to reliably transfer data, signals and/or energy from the one coupling element (4, 4', 4") to the other coupling element (5, 5', 5").

11. The coupling device (1, 1', 1") according to any one of the preceding claims, **characterized in that**
the positioning element (8, 8', 8") or the first recess (9, 9', 9") and the second recess (12) for the light source (11) are disposed on the first or second coupling element (4, 4', 4") in the opposite radial direction (28) from the common axis (10) of the two coupling elements (4, 4', 4"; 5, 5', 5").

12. The coupling device (1, 1', 1") according to any one of the preceding claims, **characterized in that**
the transfer element (14, 14', 14") and the third recess (15, 15', 15") are disposed such that they are offset by an angle (α) of preferably 55 degrees from the radial direction (28) of the second recess (12) for the light source (11) on the first and second coupling elements (4, 4', 4"; 5, 5', 5").

13. The coupling device (1, 1', 1") according to any one of the preceding claims, **characterized in that**
the positioning element (8, 8', 8"), the transfer element (14, 14', 14") and the second recess (12) for the light source (11) are disposed on the first and second coupling element (4, 4', 4"; 5, 5', 5") at the same radial distance (29, 29', 29") from the common axis (10) of the two coupling elements (4, 4', 4"; 5, 5', 5").

14. Use of a coupling device (1, 1', 1") according to any one of the preceding claims, for releasable connection of two medical, in particular dental, devices, wherein preferably at least one of these two medical, in particular dental, devices, is designed as a cleaning and/or care device, as a drive unit (3, 3', 3"), a supply tubing, a diagnostic device or as a handpiece (2, 2', 2").

15. A medical, in particular dental, device comprising a handpiece (2, 2', 2") and a drive unit (3, 3', 3") or a supply tubing, **characterized by**
a coupling device (1, 1', 1") according to any one of claims 1 - 13 for releasable connection of the handpiece (2, 2', 2") to the drive unit (3, 3', 3") or to the supply tubing.

## Revendications

1. Dispositif d'accouplement (1, 1', 1") pour la connexion amovible d'une pièce à main médicale, en particulier dentaire (2, 2', 2"), à une unité d'entraînement (3, 3', 3") ou un tuyau flexible d'alimentation pour la transmission de signaux, de données et/ou d'énergie, d'un mouvement d'entraînement, d'un fluide de travail et/ou de lumière entre la pièce à main (2, 2', 2") et l'unité d'entraînement (3, 3', 3") ou le tuyau flexible d'alimentation, dans lequel le dispositif d'accouplement (1, 1', 1") s'étend le long d'un axe longitudinal (10) et comprend: un premier et un deuxième élément d'accouplement (4, 4', 4" ; 5, 5', 5"), dans lequel l'un des deux éléments d'accouplement (5, 5', 5") est réalisé en tant qu'évidement d'accouplement (6) dans lequel une saillie d'accouplement (7) de l'autre élément d'accouplement (4, 4', 4") peut être placée, dans lequel l'évidement d'accouplement (6) et la saillie d'accouplement (7) sont respectivement entourés par une surface frontale annulaire (30, 33), un élément de positionnement (8, 8', 8") disposé au niveau de la surface frontale annulaire (30, 33) du premier ou deuxième élément d'accouplement (5, 5', 5"), lequel peut être inséré dans une première cavité (9, 9',, 9") au niveau de la surface frontale annulaire (30, 33) de l'autre élément d'accouplement (4, 4', 4") pour positionner les deux éléments d'accouplement (4, 4', 4" ; 5, 5', 5") l'un par rapport à l'autre dans une position angulaire définie autour de leur axe (10) commun, au moins une source lumineuse (11) qui est disposée dans une deuxième cavité (12) au niveau de la surface frontale annulaire (30, 33) du premier ou deuxième élément d'accouplement (4, 4', 4") et peut être couplée avec un guide de lumière (13) au niveau de la surface frontale annulaire (30, 33) de l'autre élément d'accouplement (5, 5"), ainsi qu'au moins un élément de transmission (14, 14', 14") disposé au niveau de la surface frontale annulaire (30, 33) du premier ou deuxième élément d'accouplement (4', 4" ; 5), lequel est insérable dans une troisième cavité (15, 15', 15") au niveau de la surface frontale annulaire (30, 33) de l'autre élément d'accouplement (4' ; 5', 5") pour transmettre des données, des signaux et/ou de l'énergie entre le premier et le deuxième élément d'accouplement (4, 4', 4" ; 5, 5', 5"), **caractérisé en ce que**, en vue en plan sur les surfaces frontales annulaires (30, 33) du premier et deuxième élément d'accouplement (4, 4', 4" ; 5, 5', 5"), l'élément de transmission (14, 14', 14") et la troisième cavité (15, 15', 15")
(i) sont espacés par une partie des surfaces frontales annulaires (30, 33) de l'élément de positionnement (8, 8', 8") et de la première cavité (9, 9', 9") et
(ii) sont disposés en suivant la surface frontale annulaire (30, 33) autour de l'évidement d'accouplement (6) ou la saillie d'accouplement (7) le long d'une distance radiale (29, 29', 29") par rapport à l'axe longitudinal (10) entre l'élément de positionnement (8, 8', 8") ou la première cavité (9, 9', 9") et la source lumineuse (11) ou la deuxième cavité (12) ou le guide de lumière (13).

2. Dispositif d'accouplement (1) selon la revendication 1, **caractérisé en ce que** l'élément de positionnement (8) ou la première cavité (9) présente une unité de mémoire (16) pour la mise en mémoire de données, de préférence relatives à la pièce à main, et que l'autre de ces deux éléments, à savoir l'élément de positionnement (8) ou la première cavité (9), présente une unité de lecture (17) de sorte que des données de l'unité de mémoire (16) de l'un des éléments d'accouplement (5) peuvent être transmises au moyen de l'unité de lecture (17) à l'autre élément d'accouplement (4) du dispositif d'accouplement (1).

3. Dispositif d'accouplement (1) selon la revendication 1 ou 2, **caractérisé en ce que**
l'élément de positionnement (8) et la première cavité (9) et/ou l'élément de transmission (14) et la troisième cavité (15) présentent des contacts électriques (18, 19, 20 ; 21, 22, 23) et/ou des bobines d'induction pour la transmission filaire ou sans fil de données, de signaux ou d'énergie de l'un des éléments d'accouplement (4) vers l'autre élément d'accouplement (5).

4. Dispositif d'accouplement (1) selon la revendication 3, **caractérisé en ce que**
les contacts électriques (18, 19, 20 ; 21, 22, 23) sont montés de manière élastique de sorte que ceux-ci peuvent être déplacés parallèlement à l'axe (10) ou en direction radiale (28) à l'axe (10) du dispositif d'accouplement (1).

5. Dispositif d'accouplement (1') selon l'une des revendications précédentes, **caractérisé en ce que**
l'élément de transmission (14') ou la troisième cavité (15') présente au moins un capteur (24) de sorte que des paramètres de fonctionnement de l'un des éléments d'accouplement (5'), en particulier de la pièce à main (2') ou de l'unité d'entraînement (3'), peuvent être détectés au moyen du capteur (24) et être transmis à l'autre élément d'accouplement (4') du dispositif d'accouplement (1').

6. Dispositif d'accouplement (1") selon l'une des revendications précédentes, **caractérisé en ce que**
l'élément de transmission (14") ou la troisième cavité (15") présente une caméra (25) de sorte que des données d'image, en particulier d'un tissu à traiter, peuvent être enregistrées au moyen de la caméra (25) et être transmises de l'un des éléments d'accouplement (5") à l'autre élément d'accouplement (4") du dispositif d'accouplement (1").

7. Dispositif d'accouplement (1, 1', 1") selon l'une des revendications précédentes, **caractérisé en ce que**
l'élément de positionnement (8, 8', 8") et/ou l'élément de transmission (14, 14', 14") est/sont monté(s) de manière élastique de sorte que celui-ci/ceux-ci est/sont déplaçables de préférence parallèlement à l'axe (10) du dispositif d'accouplement (1, 1', 1").

8. Dispositif d'accouplement (1, 1', 1") selon l'une des revendications précédentes, **caractérisé en ce que**
l'élément de positionnement (8, 8', 8") et/ou l'élément de transmission (14, 14', 14") est/sont réalisés de manière amovible par rapport au premier ou deuxième élément d'accouplement (4, 4', 4"; 5, 5', 5") du dispositif d'accouplement (1, 1', 1").

9. Dispositif d'accouplement (1") selon la revendication 8, **caractérisé en ce que** l'élément d'accouplement (4") présente, pour la réception amovible de l'élément de positionnement (8") et/ou de l'élément de transmission (14"), une connexion par vis, par enfichage et/ou encliquetage (26).

10. Dispositif d'accouplement (1, 1', 1") selon l'une des revendications précédentes, **caractérisé en ce que**
l'élément de positionnement (8, 8', 8") ou la première cavité (9, 9', 9"), la deuxième cavité (12) pour réceptionner la source lumineuse (11) et l'élément de transmission (14, 14', 14") ou la troisième cavité (15, 15', 15") sont disposés au niveau du premier ou deuxième élément d'accouplement (4, 4', 4"; 5, 5', 5") à distance d'une surface de base (27) à partir de laquelle la saillie d'accouplement s'étend, pour transmettre de manière sûre des données, des signaux et/ou de l'énergie de l'un des éléments d'accouplement (4, 4', 4") à l'autre élément d'accouplement (5, 5', 5").

11. Dispositif d'accouplement (1, 1', 1") selon l'une des revendications précédentes, **caractérisé en ce que**
l'élément de positionnement (8, 8', 8") ou la première cavité (9, 9', 9") et la deuxième cavité (12) pour la source lumineuse (11) sont disposés au niveau du premier ou deuxième élément d'accouplement (4, 4', 4") en direction radiale opposée (28) à l'axe commun (10) des deux éléments d'accouplement (4, 4', 4"; 5, 5', 5").

12. Dispositif d'accouplement (1, 1', 1") selon l'une des revendications précédentes, **caractérisé en ce que**
l'élément de transmission (14, 14', 14") et la troisième cavité (15, 15', 15") sont disposés de manière décalée selon un angle (a) de préférence de 55 degrés par rapport à la direction radiale (28) de la deuxième cavité (12) pour la source lumineuse (11) au niveau du premier et deuxième élément d'accouplement (4, 4', 4"; 5, 5', 5").

13. Dispositif d'accouplement (1, 1', 1") selon l'une des revendications précédentes, **caractérisé en ce que**
l'élément de positionnement (8, 8', 8"), l'élément de transmission (14, 14', 14") ainsi que la deuxième cavité (12) pour la source lumineuse (11) sont disposés au niveau du premier et deuxième élément d'accouplement (4, 4', 4"; 5, 5', 5") à une même distance radiale (29, 29', 29") par rapport à l'axe commun (10) des deux éléments d'accouplement (4, 4', 4"; 5, 5', 5").

14. Utilisation d'un dispositif d'accouplement (1, 1', 1") selon l'une des revendications précédentes pour la connexion amovible de deux dispositifs médicaux, en particulier dentaires, dans laquelle de préférence au moins l'un de ces deux dispositifs médicaux, en particulier dentaires, est réalisé en tant qu'appareil de nettoyage et/ou d'entretien, en tant qu'unité d'entraînement (3, 3', 3"), tuyau flexible d'alimentation, appareil de diagnostic ou en tant que pièce à main (2, 2', 2").

15. Dispositif médical, en particulier dentaire, comprenant une pièce à main (2, 2', 2") et une unité d'entraînement (3, 3', 3") ou un tuyau flexible d'alimentation, **caractérisé par**
un dispositif d'accouplement (1, 1', 1") selon l'une des revendications 1-13 pour la connexion amovible de la pièce à main (2, 2', 2") à l'unité d'entraînement (3, 3', 3") ou au tuyau flexible d'alimentation.
